# EUROPEAN PATENT SPECIFICATION

(11) **EP 3 110 356 B1**
(45) Date of publication and mention of the grant of the patent: **30.03.2022**
(21) Application number: 15754883.5
(22) Date of filing: 26.02.2015
(51) Int. Cl.: A61L 15/42, A61L 15/60, A61L 15/58, B32B 7/12, B32B 29/00

(54) **NOVEL ABSORBENT LAMINATE FOR DISPOSABLE ABSORBENT ARTICLES**
NEUARTIGES SAUGFÄHIGES LAMINAT FÜR SAUGFÄHIGE WEGWERFARTIKEL
NOUVEAU MATELAS ABSORBANT POUR ARTICLES ABSORBANTS JETABLES

(30) Priority: 28.02.2014 US 201461946304 P
(43) Date of publication of application: 04.01.2017
(73) Proprietor: EAM Corporation, Jesup, GA 31545 (US)
(72) Inventor: DUCKER, Paul, St. Simons Islands, GA 31522 (US); CHMIELEWSKI, Harry, Wake Forest, NC 27587 (US)
(74) Representative: Lohr, Jöstingmeier & Partner
(86) International application number: PCT/US2015/017817
(87) International publication number: WO 2015/130962

(56) References cited:
- WO-A1-98/06364
- WO-A1-2005/044163
- WO-A1-2015/171972
- US-A- 5 562 646
- US-A1- 2006 009 743
- US-A1- 2006 229 411
- US-A1- 2011 166 540
- US-A1- 2012 316 525
- None

## Description

### CROSS-REFERENCE TO RELATED APPLICATIONS

This application claims the benefit of pending U.S. Patent Application No. 61/946,304, filed on February 28, 2014.

### FIELD OF THE INVENTION

The present invention relates generally to absorbent material for use in absorbent articles and, more particularly, absorbent laminates that include particulate superabsorbent material and adhesive and that have improved properties upon storage, including improved delamination strength and liquid absorption.

### DESCRIPTION OF RELATED ART

Absorbent cores in disposable absorbent articles have gotten progressively thinner, with the amount of superabsorbent material increasing and generally pulp and other fibers decreasing. Currently, certain product offerings comprise cores that include thin absorbent laminates. These laminates comprise upper and lower layers, for example, made of tissue, with an intermediate layer sandwiched between the layers and comprising particulate superabsorbent material, possibly some fluff material, and typically a thermoplastic adhesive composition to assure the integrity of the laminate. These laminates are subjected during use to a variety of forces that can cause the laminate to separate, or delaminate. It is also observed that these products exhibit increased liquid absorption times with an increase in storage time or on store shelves. Because not all product produced and sold into the marketplace is sold and used immediately, there is a need to improve the absorption properties of the product and, particularly, the absorbent laminate upon extended storage times.

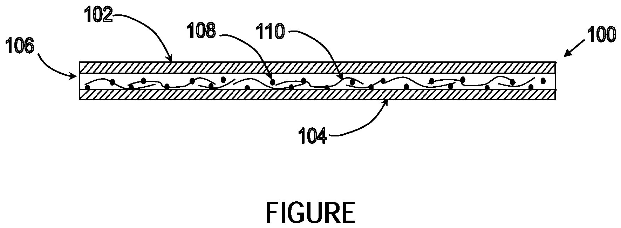

US2006/009743A relates to an absorbent article, having an absorbent core being bound to a bodyside liner, the outer cover and an intervening substrate layer. The absorbent core comprises adhesive fibers and superabsorbent material. The adhesive fibres of the embodiment depicted in the Fig.1 are of a hot melt adhesive. The glass transition temperature Tg of the adhesive is below 25°C.

WO 2015/171972 A relates to a layered absorbent structure having three substrate layers. Between the substrate layers are two liquid storage layers. The liquid storage layers each comprise a mixture of a superabsorbent polymer and hot melt adhesive.

### SUMMARY OF THE INVENTION

In accordance with one aspect of the present invention, there is provided an absorbent laminate (100) for an absorbent article, wherein the absorbent laminate includes an upper layer (102), a lower layer (104), and an intermediate layer (106), wherein
the intermediate layer is positioned between the upper and lower layers (102,104) and comprised a thermoplastic adhesive composition and a particulate superabsorbent material (108),
the adhesive composition of the intermediate layer is joined to each of the upper layer (102) and the lower layer (104), each of the upper layer (104) and the lower layer (102) is constructed of a synthetic nonwoven comprising at least one of polypropylene, polyethylene, nylon, polyester and blends of these materials, or tissue paper, wherein in case each of the upper layer (104) and the lower layer (102) is constructed of a synthetic nonwoven comprising tissue paper, the absorbent laminate consists of three layers,
the thermoplastic adhesive composition comprises a pressure-sensitive adhesive or a synthetic rubber-based pressure sensitive thermoplastic adhesive composition being selected from the group comprising styrene-butadiene-styrene (SBS) and styrene-isoprene-styrene (SIS) block copolymers,characterized in that the thermoplastic adhesive composition has a glass transition temperature of or greater than 27°C and exhibits an average rate of change in vertical delamination strength with respect to temperature in the interval from about Tg - 2°C to about Tg + 4°C that is greater than the rate of change in vertical delamination strength with respect to temperature in the interval from about Tg + 4°C to about Tg + 20°C, with the delamination strength decreasing as the temperature increases.

The thermoplastic adhesive composition can have a thermal stability sufficient to allow it to be applied at a temperature so as to yield a viscosity of about <2000 mPa.s and, in other embodiments, about <1400 mPa.s. As a further example, the thermoplastic adhesive composition can be present in the form of fibers.

Additionally, in some embodiments, the inventive absorbent laminate can exhibit a vertical delamination strength of at least about 5 N. Also, the absorbent laminate exhibits a lower 2 ml free surface absorption time upon aging at storage temperatures below Tg than for storage temperatures above Tg. These improved properties are obtained assuming the materials do not undergo other phase changes or exceed the softening point of the adhesive.

In some embodiments, at least one of the upper and lower layers, and, in some embodiments, both the upper and lower layers, are made from tissue paper. Additionally, the particulate superabsorbent material can comprise at least about 50% of the weight of the intermediate layer and, in some embodiments, greater than about 90%, and in still other embodiments, more than about 95% of the total weight. The thermoplastic adhesive composition generally can have a weight that is less than about 10% and, in other embodiments, less than about 5% of the superabsorbent weight.

In accordance with yet another aspect of the present invention, the absorbent laminate exhibits a rate of change in the 2 ml free absorption time with respect to storage time that is less for materials stored at a temperature at least about 5°C below the thermoplastic adhesive composition's glass transition temperature than for materials stored at a temperature at least about 5°C above the thermoplastic adhesive composition's glass transition temperature.

Other objects, features, and advantages of the present invention will become apparent from the following detailed description. It should be understood, however, that the detailed description and the specific examples, while indicating some embodiments of the invention, are given by way of illustration only. The present invention is defined by the claims.

### BRIEF DESCRIPTION OF THE DRAWINGS

The following drawing forms part of the present specification and is included to further demonstrate certain aspects of the present invention. The invention may be better understood by reference to the drawing in combination with the detailed description of specific embodiments presented herein. The following drawing illustrates by way of example and not limitation. For the sake of brevity and clarity, every feature of the structure may not be labeled in the figure.

The FIGURE is a schematic view of an absorbent laminate according to one embodiment of the present invention.

### DESCRIPTION OF ILLUSTRATIVE EMBODIMENTS

Various features and advantageous details are explained more fully with reference to the non-limiting embodiments that are illustrated in the accompanying drawings and detailed in the following description.

In the following description, numerous specific details are provided to present a thorough understanding of the present embodiments. One of ordinary skill in the relevant art will recognize, however, that the invention may be practiced without one or more of the specific details, or with other methods, components, materials, and so forth. In other instances, well-known structures, materials, or operations are not shown or described in detail to avoid obscuring aspects of the invention.

The terms "a" and "an" are defined as one or more unless this disclosure explicitly requires otherwise.

The term "absorbent core" means a structure positioned between a topsheet and backsheet of an absorbent article for absorbing and containing liquid received by the absorbent article and may comprise one or more substrates, absorbent polymer material, adhesives, or other materials to bind absorbent materials in the core and, for purposes of the present invention, includes the disclosed absorbent laminate.

The term "absorbent laminate" means the absorbent substrate described herein comprising top and bottom layers and an absorbent composition therebetween.

The terms "comprise" (and any form of comprise, such as "comprises" and "comprising"), "have" (and any form of have, such as "has" and "having"), "include" (and any form of include, such as "includes" and "including"), and "contain" (and any form of contain, such as "contains" and "containing") are open-ended linking verbs. As a result, a method or device that "comprises," "has," "includes," or "contains" one or more steps or elements possesses those one or more steps or elements, but is not limited to possessing only those one or more elements. Likewise, a step of a method or an element of a device that "comprises," "has," "includes," or "contains" one or more features possesses those one or more features, but is not limited to possessing only those one or more features. Furthermore, a device or structure that is configured in a certain way is configured in at least that way, but may also be configured in ways that are not listed. Metric units may be derived from the English units provided by applying a conversion and rounding to the nearest millimeter.

The term "coupled" is defined as connected, although not necessarily directly, and not necessarily mechanically. Two items are "couplable" if they can be coupled to each other. Unless the context explicitly requires otherwise, items that are couplable are also decouplable, and vice-versa. One non-limiting way in which a first structure is couplable to a second structure is for the first structure to be configured to be coupled (or configured to be couplable) to the second structure.

Terms such as "first" and "second" are used only to differentiate structures or features, and not to limit the different structures or features to a particular order.

The term "Rate of change in vertical delamination with respect to temperature" or "average rate of change in vertical delamination with respect to temperature" (used interchangeably) for a given temperature interval is defined as the absolute value of the difference between the vertical delamination values at the defined high and low temperatures of the interval, divided by the difference between the high and low temperatures and is expressed in units of Newtons per Degree Celsius.

The term "substantially" and its variations (e.g., "approximately" and "about") are defined as being largely but not necessarily wholly what is specified (and include wholly what is specified; e.g., substantially 90 degrees includes 90 degrees and substantially parallel includes parallel) as understood by one of ordinary skill in the art.

The feature or features of one embodiment may be applied to other embodiments, even though not described or illustrated, unless expressly prohibited by this disclosure or the nature of the embodiments.

Other features and associated advantages will become apparent with reference to the following detailed description of specific embodiments in connection with the accompanying drawings.

The FIGURE illustrates an embodiment of an absorbent laminate as generally applicable to the present invention. The absorbent laminate 100 comprises a first layer 102 and a second layer 104 with an intermediate layer 106 positioned there between. The intermediate layer 106 comprises a particulate superabsorbent material 108 and a thermoplastic adhesive composition 110.

First laminate layer 102 and/or lower laminate layer 104 are constructed of a synthetic nonwoven, comprising polypropylene, polyethylene, nylon, polyester and blends of these materials, or tissue paper. In some embodiments, either or both layers comprise tissue paper. For example, material 3995 or 3207 tissue from Dunn Paper, East Hartford, CT can be used.

Turning now to the intermediate layer 106, as mentioned, the intermediate layer includes particulate superabsorbent material 108. "Superabsorbent material" or "SAP" refers to water-swellable, water-insoluble material capable of absorbing many times its weight in liquid. The superabsorbent material can comprise a variety of materials, including organic compounds, such as cross-linked polymers. "Cross-linked" is a commonly understood term and refers to any approach for effectively rendering normally water-soluble materials substantially water insoluble, but swellable. Such polymers include, for example, carboxymethylcellulose, alkali metal salts of polyacrylic acids, polyacrylamides, polyvinyl ethers, hydroxypropyl cellulose, polyvinyl morpholinone, polymers and copolymers of vinyl sulfonic acid, polyacrylates, polyacrylamides, polyvinyl pyridine, and the like. Other suitable polymers include hydrolyzed acrylonitrile grafted starch, acrylic acid grafted starch, and isobutylene maleic anhydride copolymers, and mixtures thereof. Organic high-absorbency materials can include natural materials, such as agar, pectin, guar gum, and peat moss. In addition to organic materials, superabsorbent materials may also include inorganic materials, such as absorbent clays and silica gels. Examples of superabsorbent materials include W211 SAP from Nippon Shokubai, N.A.I.I., Houston, TX, and SA70 from Sumitomo Seika Chemicals Co. Ltd., Osaka, Japan.

The superabsorbent material is in particle form and can be of any desired configuration, such as granulated powders, fibers, agglomerated spheres, and other shapes known to those skilled in the art. The particle size of the superabsorbent material may vary, but typically falls between about 20 microns to about 1000 microns.

The intermediate layer of the present invention also includes a thermoplastic adhesive composition. The thermoplastic adhesive composition can be of a type that is suitable for use in the production of disposable hygiene articles and can be formulated such that it is tacky at room temperature. According to the present invention, the thermoplastic adhesive composition should have a glass transition temperature of or greater than 27°C. According to the invention, the thermoplastic adhesive composition is a thermoplastic, hot-melt adhesive composition. A thermoplastic, hot-melt adhesive composition generally comprises one or more polymers that provide cohesive strength, a tackifying resin or similar material that provides adhesive strength, possibly waxes, plasticizers, or other materials that modify viscosity, and other additives, such as antioxidants and stabilizers. According to the present invention, the thermoplastic adhesive composition comprises a pressure-sensitive, thermoplastic adhesive composition or a synthetic rubber-based pressure sensitive adhesive being selected from the group comprising a styrene-butadiene-styrene block copolymer (SBS) or a styrene-isoprene-styrene (SIS) hot melt thermoplastic adhesive composition. An example of a thermoplastic adhesive composition is SP507 adhesive from Savare Specialty Adhesives of Milan, Italy, which has a glass transition temperature of 27°C and has shown thermal stability in the viscosity ranges listed above. Another example of a thermoplastic adhesive composition is E60W adhesive also from Savare Specialty Adhesives. It too has a glass transition temperature of 27°C and has shown thermal stability in the viscosity ranges listed in the following paragraph.

In addition, the thermoplastic adhesive composition can have a thermal stability sufficient to allow it to be applied at a temperature so as to yield a viscosity of about <2000 mPa.s and, in some embodiments, about <1400 mPa.s. At these viscosities, the thermoplastic adhesive composition can be more easily attenuated into fine fibers without thermal degradation of the adhesive during handling or significant fouling of the application equipment during commercial production conditions. The amount of thermoplastic adhesive composition applied should be kept generally at the minimum amount necessary to provide a laminate with acceptable integrity.

In addition to the SAP and the adhesive, the intermediate layer may also include other materials, such as wood pulp or cellulosic fluff.

The superabsorbent material may be present in the intermediate layer in a variety of amounts, with some embodiments including the superabsorbent material as the majority component in the layer. In other embodiments, the superabsorbent material comprises at least about 90% of the total weight of the intermediate layer and, in still other embodiments, at least about 95% of the total weight of the intermediate layer. In this regard, the basis weight of the SAP in the intermediate layer may range from about 10 grams per square meter (gsm) to about 1000 gsm, such as, for example, from about 40 gsm to about 360gsm. The thermoplastic adhesive composition can comprise about 0.5% by weight to about 10% by weight of the SAP weight, such as, for example, from about 1% to about 5%. In some examples, a laminate comprises 89 gsm SAP and about 4.7% by weight (compared to the SAP weight) of thermoplastic adhesive composition. Other examples comprise 60 gsm SAP and about 5.5% by weight (compared to the SAP weight) of thermoplastic adhesive composition. Yet another example comprises 45 gsm SAP and about 6.3% by weight (compared to the SAP weight) of thermoplastic adhesive composition. Still yet another laminate comprises 82 gsm SAP and about 5.5% by weight (compared to the SAP weight) of adhesive. In a some embodiments of the absorbent laminate, each of the upper and lower layers comprise about 10 to about 30 gsm, such as between about 10 to about 20 gsm, tissue.

The absorbent laminate according to the present invention may be manufactured according to processes well known to those skilled in the art of absorbent article manufacturing. According to one such process, a roll or sheet of laminate can be made by metering a free-falling curtain of SAP particles and mixing the curtain of SAP particles with hot melt thermoplastic adhesive composition fibers.

The resulting mixture is then directed onto a moving substrate (lower layer). A second substrate (upper layer) is directed on top of the SAP-adhesive mixture to form a sandwich structure. The fibrous layer of the thermoplastic adhesive composition may form cavities in which superabsorbent material particles may reside, improving the immobilization of the particles. The fibrous thermoplastic layer may bond to the particles of the superabsorbent material, the lower laminate layer and/or the upper laminate layer. In certain embodiments, the superabsorbent material may be essentially dispersed throughout the thermoplastic adhesive composition fibers. The laminate may then be rolled up and/or cut into segments sized for use in an absorbent article.

The absorbent laminate according to the present invention exhibits improved properties and performance at temperatures below Tg for the thermoplastic adhesive composition compared to temperatures above Tg for the thermoplastic adhesive composition, including 2 ml free surface absorption times (as determined by the 2 ml Free Surface Absorption Time Test set forth later herein) upon aging and advantageous vertical delamination strength (as determined by the Vertical Delamination Strength Test set forth later herein). When Tg for the thermoplastic adhesive composition is ≥ 27°C then the material is more likely to be used and stored at temperatures below Tg and to exhibit the advantageous performance. More specifically, the inventive absorbent laminates have a vertical delamination value greater than about 5 N. A thermoplastic adhesive composition is recognized to possess discrete cohesive and adhesive properties. Without being bound to any particular theory, the adhesive properties of a thermoplastic adhesive composition are thought to promote bonding between the thermoplastic adhesive composition and the other materials in the laminate. The cohesive properties of a thermoplastic adhesive composition, particularly with respect to thermoplastic adhesive fibers, are thought to contribute to the tensile strength of the adhesive fibers against breaking in response to external forces. A thermoplastic adhesive composition demonstrating higher cohesive strength is thought to reduce the likelihood that the adhesive fibers will break and result in delamination in response to an external force compared to a thermoplastic adhesive with lower cohesive strength. This is provided that the adhesion between the glue and the other materials in the laminate exceeds that of the adhesive fibers and that the adhesion bonds do not fail first. Examples of forces that may be resisted by a thermoplastic adhesive with high cohesion include forces exerted on the laminate during converting processes that incorporate it into absorbent articles.

Additionally, the absorbent laminates according to the present invention exhibit a 2 ml free surface absorption time of less than about 15 seconds and, in some embodiments, less than about 10 seconds. The 2 ml free surface absorption time quantifies the time it takes a level sample of absorbent laminate to absorb a 2 ml drop of saline placed on the horizontal surface of the laminate under controlled conditions.

It should be noted again that, upon review of the detailed description and the drawings provided herein, it will become apparent to one of ordinary skill in the art that the present invention is also applicable to various disposable absorbent articles and, more particularly, feminine hygiene articles, such as sanitary napkins and pantiliners, to training pants, adult incontinence garments, youth pants, baby diapers, and other disposable absorbent articles that would benefit from the inclusion of an absorbent laminate of the type described herein.

### IV. Examples

The following examples are included to demonstrate some embodiments of the invention. It should be appreciated by those of skill in the art that the techniques disclosed in the examples that follow represent techniques discovered to function well in the practice of the invention and, thus, can be considered to constitute examples of its practice. However, those of skill in the art should, in light of the present disclosure, appreciate that many changes can be made in the specific embodiments which are disclosed while still obtaining a like or similar result without departing from the scope of the invention.

### Material 1:

A laminate was produced using the following materials. The upper and lower substrates were 3207 tissue from Clearwater Paper. The SAP was SA70, from Sumitomo Seika Chemicals Co. Ltd., Osaka, Japan. The thermoplastic adhesive composition was SP 507 F/ST from Savare Specialty Adhesives, having a glass transition temperature reported of 27° C. The adhesive fibers were formed by ejecting the thermoplastic adhesive composition through Omega 5,5 spray nozzles from ITW Dynatec Americas, Hendersonville, Tennessee, at a temperature of 340° F with 20 psi attenuation air. These fibers were mixed with SAP and deposited between the tissue substrates to form a sandwich with 6.2 gsm thermoplastic adhesive composition and about 80 gsm SAP and 34 gsm tissue (two tissue layers, each having a 17 gsm basis weight), to yield a material of approximately 120 gsm total basis weight.

### Material 2:

A laminate was produced in a manner similar to Material 1, except the adhesive add-on comprised 5 gsm E60W adhesive.

### Material 3:

A laminate was produced in a manner similar to Material 1, except the add-on comprised 7 gsm E60W adhesive.

### Material 4:

A laminate was produced in a manner similar to Material 1, except the add-on comprised 6 gsm E60W adhesive.

### Test 1 Delamination Strength Test

An apparatus was constructed that allowed vertical delamination measurements for laminates comprising thermoplastic adhesive compositions according to the present invention. The object of the test was to perform such measurements just below the glass transition temperature ("Tg") of the thermoplastic adhesive composition included in the intermediate layer, just above the thermoplastic adhesive composition's glass transition temperature, and then well above the thermoplastic adhesive composition's glass transition temperature. Laminates were tested to determine if vertical delamination strength is reduced with an increase in temperature and to determine if the rate of change in vertical delamination strength with respect to temperature is highest for the interval adjacent to, and that transits, Tg.

Vertical Delamination Procedure. First, a tensile tester (Zwick Z005 Tensile tester) was set up to cause compression between two parallel platens at least 2-inches in diameter. Next, a 2-inch circular sample of the inventive laminate was prepared and attached to the upper platen. In particular, a double coated tape, such as Spectape type ST 550, was used to attach the sample to the upper platen. The bottom platen was covered with a piece of 3 m double coated foam tape, or equivalent, with the release strip removed, making the surface of the lower platen adhesive with a slightly compliant surface to bond well with the irregular surface of the laminate. Subsequently, the Zwick Z005 Tensile tester was cycled as follows: the upper platen was moved toward the lower platen until the sample was compressed with a force of 35 N. The sample was then attached to both the upper and lower platens. After this force level was achieved, the upper platen was moved away from the lower platen at a rate of 75 mm/min. During the separation of the platens, the sample was delaminated. The maximum force value during the separating of the platens corresponds to the extent of bonding and is recorded as the Vertical Delamination Force, expressed in Newtons.

Temperature Control Apparatus. To analyze the effect of temperature on the vertical delamination force, an enclosure was positioned about the lower platens of the vertical delamination tester. A dryer was positioned in an opening in the enclosure and controlled such that the dryer provided a first heated air stream at a temperature equilibrium of about 47.5°C. Another dryer provided an alternative air stream at a temperature equilibrium of about 31.5°C. Ambient temperature in the laboratory was 24°C, as recorded by the digital thermometer used in this apparatus. A digital thermometer with a contact-style temperature probe was adhered to the tape on the lower platen immediately next to the sample and the temperatures were allowed to equilibrate for 30-minutes prior to testing. Samples were kept in the enclosure below the platens at the same temperature to allow equilibration.

Material 1 was tested for vertical delamination, using the above-described testing apparatus and procedure, at various temperatures and the results are recorded in the table below.

**Vertical Delamination Lab Results (N)**

| Sample/Temperature | 24°C | 31.5°C | 47.5°C |
|---|---|---|---|
| Sample 1 | 14.3 | 7.79 | 3.99 |
| Sample 2 | 10.5 | 8.48 | 4.27 |
| Sample 3 | 11.56 | 7.75 | 3.93 |
| Sample 4 | 13.75 | 9.28 | 4.9 |
| Sample 5 | 13.36 | 7.1 | 4.48 |
| **AVERAGE** | **12.7** | **8.1** | **4.3** |
| *s* | *1*.*6* | *0.8* | *0.4* |

The average vertical delamination changed 4.6 N in the interval from 24°C to 31.5°C, which transits Tg for the thermoplastic adhesive composition, yielding an average rate of change in delamination with respect to temperature of 0.62N/degree °C in this interval. The average vertical delamination changed 3.8N in the interval from 31.5°C to 47.5°C, yielding an average rate of change in delamination with respect to temperature of .24N/deg °C in this interval. Thus, the rate of change with respect to temperature was greater in the interval where Tg was transited than for an interval above Tg. When stored at 25°C or below, Material 1 takes advantage of the additional lamination strength afforded by the higher Tg.

Material 2 was tested for vertical delamination at various temperatures following the same testing procedure and apparatus as with Material 1. The Material 2 results are recorded in the table below.

**Vertical Delamination Lab Results (N)**

| S ample/Temperature | 26°C | 31°C | 47°C |
|---|---|---|---|
| Sample 1 | 9.2 | 9.69 | 4.02 |
| Sample 2 | 9.07 | 5.79 | 6.61 |
| Sample 3 | 14.44 | 7.79 | 5.38 |
| Sample 4 | 11.18 | 7.77 | 4.52 |
| Sample 5 | 12 | 8.66 | 3.52 |
| Sample 6 | | 8.29 | |
| **AVERAGE** | **11.2** | **8.0** | **4.8** |
| *s* | *2.22* | *1.29* | *1.22* |

The average vertical delamination changed 3.2 N in the interval from 26°C to 31°C, which transits Tg for the thermoplastic adhesive composition, yielding an average rate of change in delamination with respect to temperature of 0.64N/degree °C in this interval. The average vertical delamination changed 3.2N in the interval from 31°C to 47°C, yielding an average rate of change in delamination with respect to temperature of .20N/deg °C in this interval. Again, the rate of change with respect to temperature was greater in the interval where Tg was transited than for an interval above Tg. When stored at 25°C or below, Material 2 takes advantage of the additional lamination strength afforded by the higher Tg.

Material 3 was tested for vertical delamination at various temperatures following the same testing procedure and apparatus as with Material 1. The Material 3 results are recorded in the table below.

**Vertical Delamination Lab Results (N)**

| S ample/Temperature | 26°C | 31°C | 47°C |
|---|---|---|---|
| Sample 1 | 10.15 | 8.3 | 4.85 |
| Sample 2 | 12.85 | 10.01 | 4.71 |
| Sample 3 | 13.33 | 8.5 | 4.34 |
| Sample 4 | 12.15 | 7.71 | 5.43 |
| Sample 5 | 12.25 | 9.39 | 4.51 |
| Sample 6 | 15.75 | | |
| **AVERAGE** | **12.7** | **8.8** | **4.8** |
| *s* | *1.83* | *0.91* | *0.42* |

The average vertical delamination changed 3.9 N in the interval from 26°C to 31°C, which transits Tg for the thermoplastic adhesive composition, yielding an average rate of change in delamination with respect to temperature of 0.78N/degree °C in this interval. The average vertical delamination changed 4.0N in the interval from 31°C to 47°C, yielding an average rate of change in delamination with respect to temperature of .25N/deg °C in this interval. As with Materials 1 and 2, the rate of change with respect to temperature was greater in the interval where Tg was transited than for an interval above Tg. When stored at 25°C or below, Material 3 takes advantage of the additional lamination strength afforded by the higher Tg.

The absolute values for the rates of change in vertical delamination with respect to temperature across each interval for Materials 1-3 are calculated and reproduced below:

**Sample 1 Results**

| | | | |
|---|---|---|---|
| Δdelam/Δtemp 31.5C-24C | 0.62 | N/DegC | Transits Tg |
| Δdelam/Δtemp 47.5C-31.5C | 0.24 | N/Degc | |

**Sample 2 Results:**

| | | | |
|---|---|---|---|
| Δdelam/Δtemp 31C-26C | 0.64 | N/DegC | Transits Tg |
| Δdelam/Δtemp 47C-31C | 0.20 | N/DegC | |

**Sample 3 Results:**

| | | | |
|---|---|---|---|
| Δdelam/Δtemp 31C-26C | 0.79 | N/DegC | Transits Tg |
| Δdelam/Δtemp 47C-31C | 0.25 | N/DegC | |

In each case, the magnitude of the rate of change in vertical delamination with respect to temperature when the interval transited Tg was larger than when the interval was above Tg. Further, in each case, room temperature was below the Tg of these adhesives where the largest increase in vertical delamination occurs. This takes advantage of that condition to yield higher vertical delamination values for these materials.

### Test 2 2 ml Free Surface Absorbency Time Test

### Methods

This test confirmed and quantified the observation that a 2ml drop of 0.9% saline placed on a level surface of the laminate material, while generally absorbed more slowly as the laminate aged, exhibited less aging effects for the absorbent laminates of the present invention.

The 2 ml Free Surface Absorption Time Test was conducted as follows. A hand sheet of laminate material was placed flat on the lab surface. Using a Wheaton Unispense Mini (Cat 374307) peristaltic doser with a 2 mm hose ending in a barb fitting held horizontally ¼ inch above the substrate, a 2 ml drop of saline was dispensed onto the hand sheet. Using a stopwatch, the time required to absorb the drop was recorded, with the end point being that time at which the last shine from the saline solution visually disappeared form the laminate surface.

Material 1 was divided into two stacks of hand sheets. One stack was stored at room temperature (recorded to be 23-27°C by a recording device in the storage area). The other was stored in a lab oven set to 30°C. The samples were stored at their respective storage conditions and retested after 26 days in storage. The results are recorded in the tables below:

**2 ml free surface absorption after 26-day storage**

| | Samples stored at room temperature (23-27°C) | Samples stored in oven (30°C) |
|---|---|---|
| Sample 1 | 5.39 | 8.38 |
| Sample 2 | 5.03 | 7.34 |
| Sample 3 | 4.59 | 7.97 |
| Sample 4 | 5.01 | 9.03 |
| Sample 5 | 4.94 | 8.85 |
| Sample 6 | 4.66 | 8.09 |
| Sample 7 | 4.62 | 7.59 |
| Sample 8 | 5.16 | 7.01 |
| **Average** | **4.9** | **8.0** |
| *s* | *0.28* | *0.71* |

The samples stored just above the 27°C Tg for the thermoplastic adhesive composition showed a slower average absorption time than the samples stored below the 27°C Tg for the thermoplastic adhesive composition. Since a slower 2ml free surface absorption time is undesirable, storage below Tg for the thermoplastic adhesive composition is advantageous.

The experiment was repeated using Material 4. Sheets of Material 4 were tested for 2ml free surface absorption time from different hand sheets to observe the variation between hand sheets. Additionally, the 2ml free surface absorption time prior to storage was also tested in order to quantify the starting point. The results are presented below:
2ml free surface absorption time results for Material 4:

**Material 4, prior to storage**

| | Sheet 1 | Sheet 2 | Sheet 3 |
|---|---|---|---|
| Sample 1 | 5.08 | 5.3 | 4.36 |
| Sample 2 | 4.79 | 4.98 | 4.44 |
| Sample 3 | 4.52 | 5.08 | 4.61 |
| Sample 4 | 4.71 | 5.24 | 6.04 |
| Sample 5 | 4.51 | 5.17 | 5.32 |
| Sample 6 | 4.47 | 4.52 | 4.95 |
| Average | 4.7 | 5.0 | 5.0 |
| Grand Avg. 4.9 | | | |

**Material 4 after 25 days at room temperature**

| | Sheet 4 | Sheet 5 | Sheet 6 |
|---|---|---|---|
| Sample 1 | 4.23 | 6.21 | 4.71 |
| Sample 2 | 4.74 | 5.56 | 4.68 |
| Sample 3 | 4.75 | 5.34 | 4.61 |
| Sample 4 | 4.99 | 5.56 | 5.19 |
| Sample 5 | 4.48 | 5.04 | 5.24 |
| Sample 6 | 4.47 | 4.99 | 5.5 |
| Average | 4.6 | 5.5 | 5.0 |
| | Grand Avg. 5.0 | | |

**Material 4, after 25 days stored at 30°C**

| | Sheet 7 | Sheet 8 |
|---|---|---|
| Sample 1 | 6.51 | 6.1 |
| Sample 2 | 7.31 | 6.41 |
| Sample 3 | 7.14 | 6.59 |
| Sample 4 | 6.93 | 7.41 |
| Sample 5 | 6.43 | 6.75 |
| Sample 6 | 5.61 | 6.51 |
| Sample 7 | N/A | 7.03 |
| Average | 6.7 | 6.7 |
| | Grand Avg. 6.7 | |

The data again reveals that storage above Tg had a greater effect on the average 2ml free surface absorption time than storage at a temperature below Tg for the adhesive.

As these experiments show, the subject, novel laminate exhibits: (a) a greater rate of change in vertical delamination strength with respect to temperature in the temperature interval that immediately transits Tg than for temperature interval from immediately above Tg to 20°C above Tg, and (b) a greater slowing of 2ml free surface absorption time when stored above Tg than when stored below Tg at room temperature. Thus, these laminates have the beneficial effects of higher vertical delamination and less loss in absorption time when stored and used at room temperature, below the Tg of the adhesive.

The above specification and examples provide a complete description of the structure and use of exemplary embodiments. Although certain embodiments have been described above with a certain degree of particularity, or with reference to one or more individual embodiments, those skilled in the art could make numerous alterations to the disclosed embodiments without departing from the scope of this invention. As such, the various illustrative embodiments of the present structures and methods are not intended to be limited to the particular forms disclosed. Rather, they include all modifications and alternatives falling within the scope of the claims, and embodiments other than the ones shown or described may include some or all of the features of the depicted or described embodiments. For example, components may be combined as a unitary structure and/or connections may be substituted. Further, where appropriate, aspects of any of the examples described above may be combined with aspects of any of the other examples depicted or described to form further examples having comparable or different properties and addressing the same or different problems. Similarly, it will be understood that the benefits and advantages described above may relate to one embodiment or may relate to several embodiments.

## Claims

1. An absorbent laminate (100) for an absorbent article, wherein the absorbent laminate includes an upper layer (102), a lower layer (104), and an intermediate layer (106), wherein
- the intermediate layer is positioned between the upper and lower layers (102, 104) and comprised a thermoplastic adhesive composition and a particulate superabsorbent material (108),
- the adhesive composition of the intermediate layer is joined to each of the upper layer (102) and the lower layer (104),
- each of the upper layer (102) and the lower layer (104) is constructed of a synthetic nonwoven comprising at least one of polypropylene, polyethylene, nylon, polyester and blends of these materials, or tissue paper, wherein in case each of the upper layer (102) and the lower layer (104) is constructed of a synthetic nonwoven comprising tissue paper, the absorbent laminate consists of three layers,
- the thermoplastic adhesive composition comprises a pressure-sensitive adhesive or a synthetic rubber-based pressure sensitive thermoplastic adhesive composition being selected from the group comprising styrene-butadiene-styrene (SBS) and styrene-isoprene-styrene (SIS) block copolymers,
**characterized in that**
the thermoplastic adhesive composition has a glass transition temperature ("Tg") of or greater than 27°C (Tg≥27°C), and exhibits an average rate of change in vertical delamination strength with respect to temperature in the interval from Tg -2°C to Tg + 4°C that is greater than the rate of change in vertical delamination strength with respect to temperature in the interval from Tg + 4°C to Tg + 20°C, with the vertical delamination strength decreasing as the temperature increases.

2. An absorbent laminate as claimed in claim 1, **characterized in that** the thermoplastic adhesive composition comprises fibers.

3. An absorbent laminate as claimed in claim 1, **characterized in that** the thermoplastic adhesive composition is thermally stable at temperatures that yield a viscosity below 2000 mPa·s or below 1400 mPa·s.

4. An absorbent laminate as claimed in claim 1, **characterized in that** at least one of the upper layer and/or the lower layer comprises tissue paper.

5. An absorbent laminate as claimed in claim 1, **characterized in that** the weight of thermoplastic adhesive composition is less than 5% of the weight of the superabsorbent material.

6. An absorbent laminate as claimed in claim 1, **characterized in that** the particulate superabsorbent material and the thermoplastic adhesive composition are formed as a mixture.

## Patentansprüche

1. Ein absorbierendes Laminat (100) für einen absorbierenden Artikel, wobei das absorbierende Laminat eine obere Schicht (102), eine untere Schicht (104), und eine Zwischenschicht (106) aufweist, wobei
- die Zwischenschicht zwischen der oberen und der unteren Schicht (102, 104) angeordnet ist und eine thermoplastische Klebstoffzusammensetzung und ein teilchenförmiges superabsorbierendes Material aufweist,
- die Klebstoffzusammensetzung der Zwischenschicht zu sowohl der oberen Schicht (102) als auch der unteren Schicht (104) verbunden ist,
- sowohl die obere Schicht (102) als auch der untere Schicht (104) aus einem synthetischen Vlies gebildet sind, welches mindestens eines von Polypropylen, Polyethylen, Nylon, Polyester und Mischungen dieser Materialien, oder Gewebepapier aufweist, wobei falls die obere Schicht (102) und die untere Schicht (104) jeweils aus einem synthetischen Vlies gebildet sind, welches Gewebepapier aufweist, das absorbierende Laminat aus drei Schichten besteht,
- die thermoplastische Klebstoffzusammensetzung einen druckempfindlichen Klebstoff oder eine synthetische kautschukbasierte druckempfindliche thermoplastische Klebstoffzusammensetzung aufweist, welche aus der Gruppe ausgewählt ist bestehend aus Styren-Butadien-Styren (SBS) und Styren-Isopren-Styren (SIS) Blockcopolymeren,
**dadurch gekennzeichnet, dass**
die thermoplastische Klebstoffzusammensetzung eine Glasübergangstemperatur ("Tg") von oder von mehr als etwa 27°C (Tg ≥ 27°C) hat, und eine durchschnittliche Änderungsrate in der vertikalen Delaminationsstärke in Bezug auf die Temperatur im Intervall von Tg - 2 °C bis Tg + 4 °C zeigt, die größer ist als die Änderungsrate der vertikalen Delaminationsstärke in Bezug auf die Temperatur im Intervall von Tg +4°C bis Tg +20°C, wobei die vertikale Delaminationsstärke mit steigender Temperatur abnimmt.

2. Ein absorbierendes Laminat wie in Anspruch 1 beansprucht, **dadurch gekennzeichnet, dass** die thermoplastische Klebstoffzusammensetzung Fasern aufweist.

3. Ein absorbierendes Laminat wie in Anspruch 1 beansprucht, **dadurch gekennzeichnet, dass** die thermoplastische Klebstoffzusammensetzung thermisch stabil bei Temperaturen ist, die eine Viskosität unter 2000 mPa·s oder unter 1400 mPa·s ergeben.

4. Ein absorbierendes Laminat wie in Anspruch 1 beansprucht, **dadurch gekennzeichnet, dass** mindestens eine von der oberen Schicht und/oder der unteren Schicht Gewebepapier aufweist.

5. Ein absorbierendes Laminat wie in Anspruch 1 beansprucht, **dadurch gekennzeichnet, dass** pdas Gewicht der thermoplastischen Klebstoffzusammensetzung weniger als 5% des Gewichts des superabsorbierenden Materials ist.

6. Ein absorbierendes Laminat wie in Anspruch 1 beansprucht, **dadurch gekennzeichnet, dass** das teilchenförmige superabsorbierende Material und die thermoplastische Klebstoffzusammensetzung als ein Gemisch geformt sind.

## Revendications

1. Stratifié absorbant (100) pour un article absorbant, dans lequel le stratifié absorbant comporte une couche supérieure (102), une couche inférieure (104), et une couche intermédiaire (106), dans lequel
- la couche intermédiaire est positionnée entre les couches supérieure et inférieure (102, 104) et composée d'une composition adhésive thermoplastique et d'un matériau superabsorbant à particules (108),
- la composition adhésive de la couche intermédiaire est jointe à chacune de la couche supérieure (102) et de la couche inférieure (104),
- chacune de la couche supérieure (102) et de la couche inférieure (104) est construite à partir d'un non tissé synthétique comprenant au moins l'un parmi le polypropylène, le polyéthylène, le nylon, le polyester et des mélanges de ces matériaux, ou du papier mince, dans lequel dans le cas où chacune de la couche supérieure (102) et de la couche inférieure (104) est construite à partir d'un non tissé synthétique comprenant du papier mince, le stratifié absorbant est constitué de trois couches,
- la composition adhésive thermoplastique comprend un adhésif autocollant ou une composition adhésive thermoplastique autocollante à base de caoutchouc synthétique qui est choisi dans le groupe comprenant des copolymères séquencés de styrène-butadiène-styrène (SBS) et de styrène-isoprène-styrène (SIS),
**caractérisé en ce que**
la composition adhésive thermoplastique a une température de transition vitreuse (« Tg ») de 27 °C ou plus (Tg ≥ 27 °C), et présente un taux de variation moyen de résistance à la déstratification verticale vis-à-vis de la température dans l'intervalle de Tg -2 °C à Tg +4 °C qui est supérieur au taux de variation de résistance à la déstratification verticale vis-à-vis de la température dans l'intervalle Tg +4 °C à Tg +20 °C, la résistance à la déstratification verticale augmentant à mesure que la température diminue.

2. Stratifié absorbant selon la revendication 1, **caractérisé en ce que** la composition adhésive thermoplastique comprend des fibres.

3. Stratifié absorbant selon la revendication 1, **caractérisé en ce que** la composition adhésive thermoplastique est thermiquement stable à des températures qui donnent une viscosité inférieure à 2 000 mPa.s ou inférieure à 1 400 mPa.s.

4. Stratifié absorbant selon la revendication 1, **caractérisé en ce qu'**au moins l'une de la couche supérieure et/ou de la couche inférieure comprend du papier mince.

5. Stratifié absorbant selon la revendication 1, caractérisé en ce le poids de la composition adhésive thermoplastique est inférieur à 5 % du poids du matériau superabsorbant.

6. Stratifié absorbant selon la revendication 1, **caractérisé en ce que** le matériau superabsorbant à particules et la composition adhésive thermoplastique sont formés en mélange.
